(19) Europäisches Patentamt
European Patent Office
Office européen des brevets

(11) Veröffentlichungsnummer: **0 112 550**
**B1**

# (12) EUROPÄISCHE PATENTSCHRIFT

(45) Veröffentlichungstag der Patentschrift: **07.02.90**

(51) Int. Cl.[5]: **C 07 D 501/46**, A 61 K 31/545

(21) Anmeldenummer: **83112862.4**

(22) Anmeldetag: **21.12.83**

## (54) Kristallisierte Cephem-Säureadditionssalze und Verfahren zu ihrer Herstellung.

(30) Priorität: **28.12.82 DE 3248281**

(43) Veröffentlichungstag der Anmeldung:
**04.07.84 Patentblatt 84/27**

(45) Bekanntmachung des Hinweises auf die Patenterteilung:
**07.02.90 Patentblatt 90/06**

(84) Benannte Vertragsstaaten:
**AT BE CH DE FR GB IT LI LU NL SE**

(56) Entgegenhaltungen:
**EP-A-0 064 740**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT**
**Postfach 80 03 20**
**D-6230 Frankfurt am Main 80 (DE)**

(72) Erfinder: **Dürckheimer, Walter, Dr.**
**Im Lerchenfeld 45**
**D-6234 Hattersheim am Main (DE)**
Erfinder: **Lattrell, Rudolf, Dr.**
**Heuhohlweg 6h**
**D-6240 Königstein/Taunus (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 112 550 B1

LIBERGRAF, STOCKHOLM 1990

**Beschreibung**

Gegenstand der Erfindung sind ein kristallisiertes Cephem-Säureadditionssalz der Formel I, dessen Hydrate, sowie ein Verfahren zu seiner Herstellung.

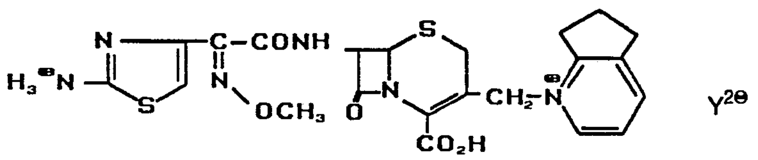

(I)

In der Formel I bedeutet $Y^{2\ominus}$ das Anion $SO_4^{2\ominus}$.

Das Sulfat ist für die parenterale Anwendung besonders geeignet, da es sich durch eine sehr geringe Tendenz des Einbaus organischer Lösungsmittel in das Kristallgitter auszeichnet.

Das Verfahren zur Herstellung des Säureadditionssalzes der Formel I ist dadurch gekennzeichnet, daß man ein Cephem-Betain der Formel II

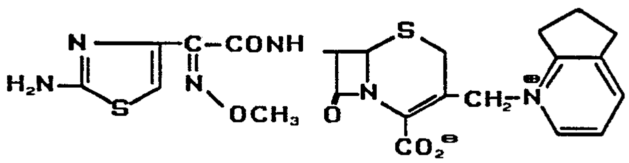

(II)

mit einer Säure der Formel III

$$2H^{\oplus}Y^{2\ominus} \qquad \text{(III)}$$

worin $Y^{2\ominus}$ die vorstehend genannte Bedeutung besitzt, umsetzt.

Die Herstellung des dem erfindungsgemäßen Salz der Formel I zugrundeliegenden Betains der Formel II wird in der EP-A-0 064 740 beschrieben. Dort werden auch bestimmte, amorph anfallende Säureadditionssalze erwähnt.

Nach dem erfindungsgemäßen Verfahren wird eine Lösung der Verbindung II in Wasser oder in einem Gemisch aus Wasser und einem mit Wasser mischbaren organischen Lösungsmittel, wie z. B. Methanol, Äthanol, Isopropanol, Aceton, Tetrahydrofuran oder Acetonitril, mit einer Säure der Formel III versetzt. Die Säure $2H^{\oplus}Y^{2\ominus}$ kann in Substanz oder in Lösung, beispielsweise in Wasser oder in mit Wasser mischbaren, vorstehend genannten organischen Lösungsmitteln, oder in Gemischen aus Wasser und diesen Lösungsmitteln zugegeben werden.

Die Bildung des Salzes der Formel I wird bei Temperaturen zwischen -20° und +80°C, vorzugsweise zwischen -5° und +30°C vorgenommen. Die Kristallisation des Salzes erfolgt spontan beim Stehen, unter Rühren oder nach Animpfen oder durch Ausfällen mit einem Lösungsmittel wie Aceton oder Äthanol.

Die Säure der Formel III muß in mindestens doppelt äquimolarer Menge zugefügt werden, jedoch kann auch ein Überschuß eingesetzt werden.

Nach Zugabe der Säure III bildet sich zunächst eine Lösung, die filtriert werden kann, wobei es in manchen Fällen von Vorteil sein kann, vor der Filtration mit Aktivkohle zu klären.

Das Verfahren kann auch so durchgeführt werden, daß man die Säure III gelöst vorlegt und die Cephembase II direkt oder in Wasser gelöst, zugibt.

Die Ausgangsverbindung der Formel II kann auch aus einem ihrer Salze durch Behandeln mit einem basischen Ionenaustauscher, z. B. dem Festaustauscher Amberlite IRA 93 oder dem Flüssigaustauscher Amberlite LA-2, in wäßriger Lösung freigesetzt werden und dann wie vorstehend in das Säureadditionssalz überführt werden.

Die nach den vorstehenden Verfahren erhaltene Verbindung der Formel I wird beispielsweise durch Filtration oder Zentrifugieren isoliert und zweckmäßigerweise mit Hilfe eines wasserentziehenden Mittels, wie z. B. konz. Schwefelsäure oder Phosphorpentoxid, bei Normaldruck oder im Vakuum getrocknet. Hierbei fällt die Verbindung der Formel I in Abhängigkeit von den Trocknungsbedingungen als Hydrat oder wasserfrei an.

Die erfindungsgemäß erhaltene Verbindung der Formel I zeigt bemerkenswert gute antibakterielle Wirksamkeiten, sowohl gegen grampositive als auch gramnegative bakterielle Keime.

Auch gegen penicillinase- und cephalosporinase-bildende Bakterien ist die Verbindung der Formel I unerwartet gut wirksam. Da sie zudem günstige toxikologische und pharmakologische Eigenschaften zeigt, stellt sie ein wertvolles Chemotherapeutikum dar.

Die Erfindung betrifft somit auch Arzneipräparate zur Behandlung von mikrobiellen Infektionen, die durch einen Gehalt an der erfindungsgemäßen Verbindung charakterisiert sind.

einen Gehalt an der erfindungsgemäßen Verbindung charakterisiert sind.

Das erfindungsgemäße Produkt kann auch in Kombination mit anderen Wirkstoffen, beispielsweise aus der Reihe der Penicilline, Cephalosporine oder Aminoglykoside zur Anwendung kommen.

Die Verbindung der Formel I kann subcutan, intramuskulär, intraarteriell oder intravenös verabfolgt werden.

Arzneipräparate, die die Verbindung der Formel I als Wirkstoff enthalten, können hergestellt werden, indem man die Verbindung der Formel I mit einem oder mehreren pharmakologisch verträglichen Trägerstoffen oder Verdünnungsmittel, wie z. B. Puffersubstanzen, vermischtund in eine zur parenteralen Applikation geeignete Zubereitungsform bringt.

Als Verdünnungsmittel seien beispielsweise erwähnt Polyglykole, Äthanol und Wasser. Puffersubstanzen sind beispielsweise organische Verbindungen, wie z. B. N;N'-Dibenzyläthylendiamin, Diäthanolamin, Äthylendiamin, N-Methylglucamin, N-Benzylphenäthylamin, Diäthylamin, Tris-(hydroxymethyl)aminomethan, oder anorganische Verbindungen, wie z. B. Phosphatpuffer, Natriumbicarbonat, Natriumcarbonat. Für die parenterale Applikation kommen vorzugsweise Suspensionen oder Lösungen in Wasser mit oder ohne Puffersubstanzen in Betracht.

Geeignete Dosen der Verbindung der Formel I liegen bei etwa 0,4 bis 20 g/Tag, vorzugsweise bei 0,5 bis 4 g/Tag, für einen Erwachsenen von etwa 60 kg Körpergewicht.

Es können Einzel- oder im allgemeinen Mehrfachdosen verabreicht werden, wobei die Einzeldosis den Wirkstoff in einer Menge von etwa 50 bis 1000 mg vorzugsweise von etwa 100 bis 500 mg enthalten kann.

Es war überraschend, daß erfindungsgemäß kristalline Salze mit erhöhter Stabilität entstehen.

Die folgenden Ausführungsbeispiele für die erfindungsgemäß herstellbare Säureadditionsverbindung der Verbindung II, dem 1-[[(6R,7R)-7-]2-(2-Amino-4-thiazolyl)-glyoxylamido]-2-carboxy-8-oxo-5-thia-1-azabicyclo[4.2.0]oct-2-en-3-yl]methyl]-6,7-dihydro-5H-cyclopenta[b]pyridiniumhydroxid, inneres Salz, $7^2$-(Z)-(O-methyloxim), dienen zur weiteren Erläuterung der Erfindung.

**Referenzbeispiel**

**Dihydronitrat der Verbindung II**

10.3 g (0.02 Mol) der Verbindung II löst man in 80 ml Wasser bei Raumtemperatur und gibt unter Umschütteln auf einmal 100 ml 0.85 n Salpetersäure zu. Die unlöslichen Anteile werden sofort abgesaugt und mit 10 ml 0.85 n Salpetersäure gewaschen. Aus dem Filtrat kristallisiert innerhalb von 15 Minuten das Dihydronitrat aus. Nach 2 Stdn. Stehen bei 5°C wird abgesaugt, mit 20 ml 0.85 n Salpetersäure und dreimal mit je 50 ml Diäthyläther gewaschen. Nach dem Trocknen bei Raumtemperatur im Vakuum über $P_2O_5$ erhält man 9.8 g (72.5 % d. Th.) farblose Kristalle, Zersetzungspunkt 138 - 140°C. Durch Einengen der Mutterlauge im Vakuum lassen sich weitere 0.9 g (6.6 %) von Zers.P. 134°C gewinnen

$C_{22}H_{22}N_6O_5S_2$ x $2HNO_3$ x $2H_2O$ (676.65)

| | | | | |
|---|---|---|---|---|
| Ber. C 39.05 | H 4.17 | N 16.56 | S 9.48 | $H_2O$ 5.32 % |
| Gef. C 38.9 | H 4.2 | N 16.6 | S 9.4 | $H_2O$ 5.3 % |

$^1$H-NMR($CF_3CO_2D$): δ = 2.30 - 2.85 (m, 2H, Cyclopenten-H); 3.15 - 4.05 (m, 6H, 4-Cyclopenten-H und $SCH_2$); 4.30 (s,30 (s, 3H, $OCH_3$); 5.20 - 6.35 (m, 4H, $CH_2$Py und 2 Lactam-H); 7.65 - 8.72 (m, 3H, Py); 9.22 ppm (s, 1H, Thiazol)

**Beispiel 1**

**Sulfat der Verbindung II**

25.7 g (0.05 Mol) der Verbindung II werden in 80 ml Wasser gelöst und unter Rühren bei 5°C 100 ml 1 n Schwefelsäure und sodann 250 ml Äthanol zugetropft. Geringe harzige Verunreinigungen werden gegebenenfalls über ein Klärschichtfilter abgesaugt. Aus dem klaren Filtrat kristallisiert innerhalb von ca. 1 Stunde das Sulfat aus. Nach 3 Stunden wird abgesaugt, mit 40 ml Wasser: Äthanol (1 : 2) sowie zweimal mit je 30 ml Äthanol gewaschen. Nach dreitägigem Trocknen über Schwefelsäure bei Normaldruck erhält man 24.6 g (78 % d. Th.) farblose Kristalle, Zers. bei 198 - 202°C.

($C_{22}H_{22}N_6O_5S_2$ x $H_2SO_4$ x $H_2O$ (630.70)

| | | | | |
|---|---|---|---|---|
| Ber. C 41.90 | H 4.16 | N 13.33 | S 15.25 | $H_2O$ 2.85 % |
| Gef. C 41.9 | H 4.2 | N 13.4 | S 15.0 | $H_2O$ 2.3 % |

$^1$H-NMR ($D_2O$): δ = 2.00 - 2.60 (m, 2H, Cyclopenten-H); 3.00 - 3.70 (m, 6H, 4 Cyclopenten-H und $SCH_2$); 4.03 (s, 3H, $OCH_3$); 5.15 - 5.93 (m, 4H, $CH_2$Py und 2 Lactam-H); 7.11 (s, 1H, Thiazol); 7.66 - 8.62ppm (m, 3H, Py)

**Beispiel 2**

**Sulfat der Verbindung II**

33.7 g (0.05 Mol) Dihydronitrat, erhalten nach Referenzbeispiel, werden in 100 ml Wasser suspendiert, eine Lösung von 50 ml Amberlite LA-2 (Serva 40610) in 200 ml Diäthyläther zugegeben und die Mischung 15 Minuten im Eisbad gerührt. Die Phasen werden getrennt und die Wasserphase viermal mit je 100 ml Methylendichlorid extrahiert. Zur Wasserphase werden unter Rühren im Eisbad 100 ml 1 n Schwefelsäure und sodann 350 ml Äthanol getropft. Geringe ungelöste harzige Anteile werden durch Filtration über eine Klärschicht entfernt. Aus dem Filtrat kristallisiert das Sulfat aus. Nach 2 Stunden im Eisbad wird abgesaugt, mit Alkohol gewaschen und wie oben getrocknet. Die Ausbeute beträgt 18.9 g (59.9 % d. Th.). Die Verbindung ist in allen Eigenschaften mit der vorstehend beschriebenen identisch.

**Patentansprüche** für die Vertragsstaaten: BE, CH, DE, FR, GB, IT, LI, LU, NL, SE

1. Kristallisiertes Cephem-Säureadditionssalz der Formel I und dessen Hydrate

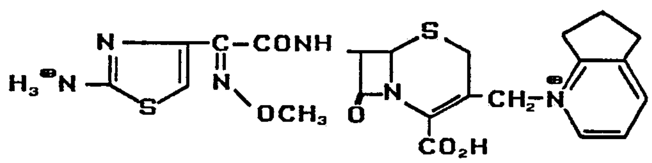 $Y^{2\ominus}$ (I)

worin
$Y^{2\ominus}$ für das Anion $SO_4^{2\ominus}$ steht.

2. Verfahren zur Herstellung des Säureadditionssalzes der Formel I, dadurch gekennzeichnet, daß man ein Cephem-Betain der Formel II

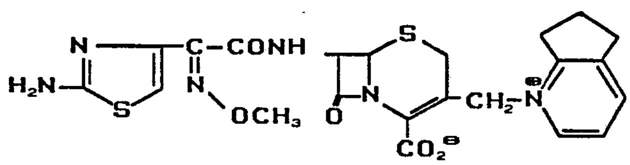 (II)

mit einer Säure der Formel III

$$2H^{\oplus}Y^{2\ominus} \quad (III)$$

worin $Y^{2\ominus}$ die vorstehende Bedeutung besitzt, umsetzt.

3. Gegen bakterielle Infektionen wirksame pharmazeutische Präparate, gekennzeichnet durch einen Gehalt an dem Cephemderivat der Formel I.

4. Verfahren zur Herstellung von gegen bakterielle Infektionen wirksamen pharmazeutischen Präparaten, dadurch gekennzeichnet, daß das Cephemderivat der Formel I gegebenenfalls mit pharmazeutisch üblichen Trägerstoffen, Verdünnungsmitteln oder Puffersubstanzen in eine pharmazeutisch geeignete Verabreichungsform gebracht wird.

5. Cephemderivat der Formel I zur Bekämpfung bakterieller Infektionen.

**Patentanspruch** für den Vertragsstaat: AT

1. Verfahren zur Herstellung des kristallisierten Cephem-Säureadditionssalzes der Formel I

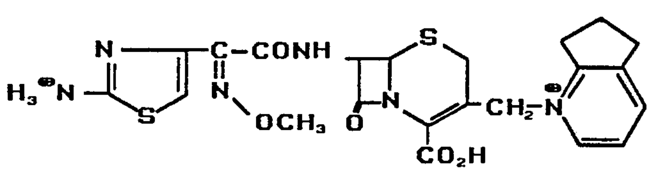 $Y^{2\ominus}$ (I)

und von dessen Hydraten,
worin
$Y^{2\ominus}$ für das Anion $SO_4^{2\ominus}$ steht, dadurch gekennzeichnet, daß man ein Cephem-Betain der Formel II

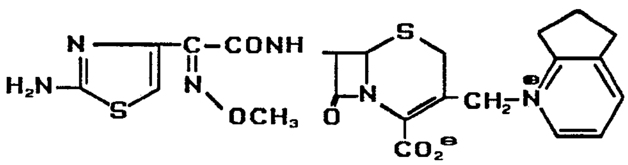

(II)

mit einer Säure der Formel III

$2H^{\oplus}Y^{2\ominus}$ (III)

worin $Y^{2\ominus}$ die vorstehende Bedeutung besitzt, umsetzt.

**Claims** for the Contracting States: BE, CH, DE, FR, GB, IT, LI, LU, NL, SE

1. A crystalline cephem-acid addition salt of the formula I or a hydrate thereof

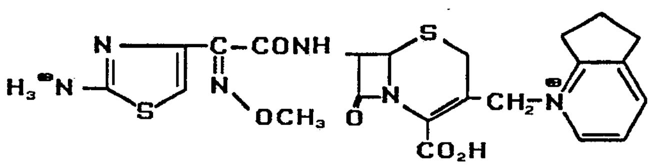

$Y^{2\ominus}$ (I)

in which
$Y^{2\ominus}$ represents the anion $SO_4^{2\ominus}$.

2. A process for the preparation of an acid addition salt of the formula I, which comprises reacting a cephembetaine of the formula II

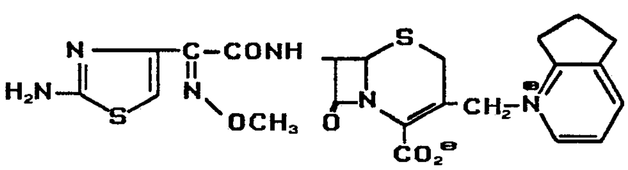

(II)

with acid of the formula III

$2H^{\oplus}Y^{2\ominus}$ (III)

in which $Y^{2\ominus}$ has the above meaning.

3. A pharmaceutical product which is effective against bacterial infections, which contains cephem derivatives of the formula I.

4. A process for the preparation of a pharmaceutical product which is effective against bacterial infections, which comprises converting the cephem derivative of the formula I into a pharmaceutically suitable form of administration, if necessary with pharmaceutically conventional excipients, diluents or buffer substances.

5. A cephem derivative of the formula I for combating bacterial infections.

**Claims** for the Contracting State: AT

1. A process for the preparation of crystalline cephem-acid addition salt of the formula I

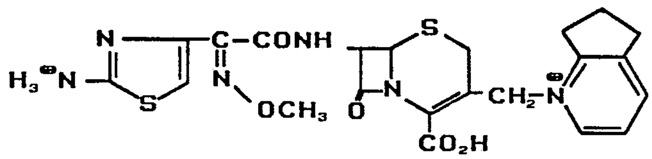

$Y^{2\ominus}$ (I)

in which $Y^{2\ominus}$ represents the anion $SO_4^{2\ominus}$. which comprises reacting a cephem-betaine of the formula II

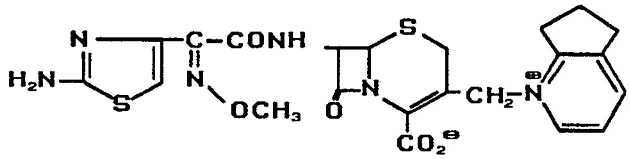

(II)

with acid of the formula III

$2H^{\oplus}Y^{2\ominus}$ (III)

in which $Y^{2\ominus}$ has the above meaning.

**Revendications** pour les Etats Contractants: BE, CH, DE, FR, GB, IT, LI, LU, NL, SE

1. Sel d'addition à un acide, cristallisé, d'un dérivé céphem, de formule I

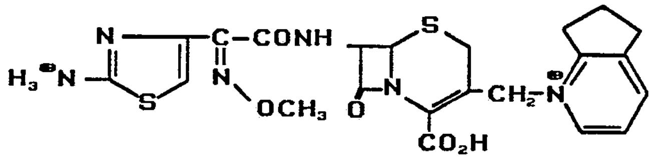

$Y^{2\ominus}$ (I)

dans laquelle $Y^{2\ominus}$ représente l'anion $SO_4^{2\ominus}$ et ses hydrates.

2. Procédé de préparation du sel d'addition à un acide, de formule I, caractérisé en ce que l'on fait réagir un céphem-bétaïne de formule II

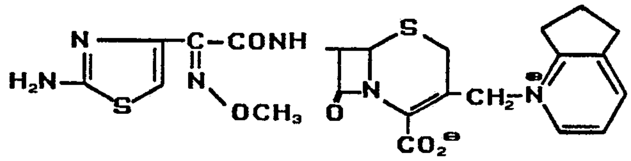

(II)

avec un acide de formule III

$2H^{\oplus}Y^{2\ominus}$ (III)

dans laquelle $Y^{2\ominus}$ a la signification ci-dessus.

3. Préparations pharmaceutiques efficaces contre les infections bactériennes, caractérisées en ce qu'elles contiennent un dérivé de céphem de formule I.

4. Procédé de fabrication de préparations pharmaceutiques efficaces contre les infections bactériennes, caractérisé en ce que le dérivé de céphalosporine de formule I, éventuellement avec des excipients pharmaceutiques classiques, des diluants ou des substances tampons, est amené à une forme d'administration pharmaceutiquement appropriée.

5. Dérivé de céphalosporine de formule I destiné à combattre des infections bactériennes.

**Revendications** l'Etat Contractant: AT

Procédé de préparation du sel d'addition à un acide, cristallisé, d'un dérivé céphem, de formule générale I

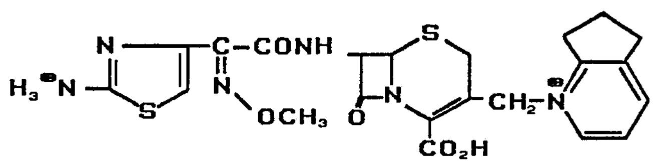 $Y^{2\ominus}$ (I)

dans laquelle $Y^{2\ominus}$ représente l'anion $SO_4^{2\ominus}$ et de ses hydrates, caractérisé en ce que l'on fait réagir un céphem-bétaïne de formule II

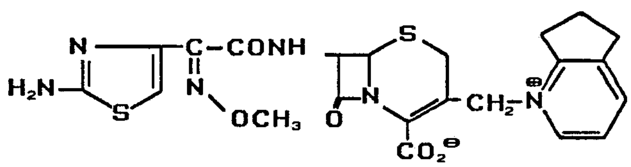 (II)

avec un acide de formule III

$2H^{\oplus}Y^{2\ominus}$ (III)

dans laquelle $Y^{2\ominus}$ a la signification ci-dessus.